# EUROPÄISCHE PATENTSCHRIFT

(11) **EP 0 866 672 B1**
(45) Veröffentlichungstag und Bekanntmachung des Hinweises auf die Patenterteilung: **12.09.2001**
(21) Anmeldenummer: 96945879.3
(22) Anmeldetag: 07.11.1996
(51) Int. Cl.: A61B 17/00

(54) **ANORDNUNG ZUR ELEKTRO-THERMISCHEN BEHANDLUNG DES MENSCHLICHEN ODER TIERISCHEN KÖRPERS**
ARRANGEMENT FOR ELECTROTHERMAL TREATMENT OF THE HUMAN OR ANIMAL BODY
SYSTEME DE TRAITEMENT ELECTROTHERMIQUE DU CORPS HUMAIN OU ANIMAL

(30) Priorität: 08.11.1995 DE 19541566
(43) Veröffentlichungstag der Anmeldung: 30.09.1998
(60) Teilanmeldung: 00250244.1
(73) Patentinhaber: Celon AG Medical Instruments, 14513 Teltow (DE)
(72) Erfinder: MÜLLER, Gerhard, D-14129 Berlin (DE); DESINGER, Kai, D-10827 Berlin (DE)
(74) Vertreter: Eisenführ, Speiser & Partner
(86) Internationale Anmeldenummer: DE9602164
(87) Internationale Veröffentlichungsnummer: WO9717009

(56) Entgegenhaltungen:
- EP-A- 0 530 400
- WO-A-92/19167
- WO-A-94/02077
- WO-A-95/10320
- DE-A- 3 930 451
- US-A- 4 565 200
- US-A- 4 832 048
- US-A- 5 007 908

## Beschreibung

Die Erfindung betrifft eine Anordnung zur elektrothermischen Behandlung des menschlichen oder tierischen Körpers, insbesondere zur Elektrokoagulation oder Elektrotomie, gemäß dem Oberbegriff des Anspruchs 1.

Es ist seit längerem in der Chirurgie bekannt, zur Gewebekoagulation und zur Gewebetrennung hochfrequente Wechselströme im Frequenzbereich von 300 kHz bis 2 MHz zu verwenden, wodurch das behandelte Gewebe koaguliert bzw. vaporisiert wird, was als Elektrokoagulation bzw. Elektrotomie bezeichnet wird. Hierbei ist zu unterscheiden zwischen der monopolaren und der bipolaren HF-Thermotherapie.

Bei der monopolaren HF-Thermotherapie wird eine Elektrode - auch als Neutralelektrode bezeichnet - als großflächige Patientenableitung ausgelegt und in der Nähe der Eingriffsstelle am Patienten angebracht. Die eigentliche Arbeitselektrode - auch als Aktivelektrode bezeichnet - ist in ihrer Form an die jeweilige Anwendung angepaßt. So werden zur Gewebekoagulation großflächige Kugel-, Platten- oder Nadelelektroden verwendet, während bei der Gewebetrennung dünne Lanzetten- oder Schlingenelektroden eingesetzt werden.

Bei der bipolaren HF-Thermotherapie hingegen sind beide Elektroden in unmittelbarer Nähe zur Eingriffsstelle angeordnet, so daß die Wirkung des Wechselstroms auf die unmittelbare Umgebung der Eingriffsstelle begrenzt ist, wodurch ein hohes Maß an Sicherheit für Patient und Anwender gegeben ist, de Unfälle durch kapazitive Leckströme oder Verbrennung an der Neutralelektrode nicht mehr auftreten können. Ein weiterer Vorteil der bipolaren HF-Thermotherapie besteht in dem wesentlich geringeren Lastwiderstand des Gewebes zwischen den beiden Elektroden, was unter Beibehaltung des thermischen Effekts eine Verringerung der erforderlichen Generatorleistung ermöglicht.

Die HF-Thermotherapie läßt sich weiterhin einteilen nach der Lage der Elektroden in die Oberflächenkoagulation einerseits und die Tiefenkoagulation andererseits.

Bei der Oberflächenkoagulation werden in der bipolaren Technik zwei parallel angeordnete Tastelektroden verwendet, die auf die Gewebeoberfläche aufgesetzt werden, wodurch das darunterliegende Gewebe infolge des Stromflusses erhitzt und damit koaguliert wird.

Bei der Tiefenkoagulation ist es zur monopolaren Elektrotomie bekannt, Nadel-, Lanzetten- oder Schlingenelektroden zu verwenden. Hierbei müssen an der Aktivelektrode elektrische Lichtbögen generiert werden, um das vor der Aktivelektrode liegende Gewebe zu vaporisieren und damit einen Gewebsschnitt zu realisieren. Bei der monopolaren Technik ist dies verhältnismäßig einfach, da es hier nur einer bestimmten Feldstärke bedarf, um an der Aktivelektrode einen Funkenüberschlag auszulösen. Die bipolare Technik stellt hier größere Anforderungen an die Konzeption der Elektrodenkonfiguration, da die physikalischen Vorgänge hierbei nicht einfach zu beherrschen sind. Es sind deshalb nur wenige bipolare Elektrodenanordnungen für die Tiefenkoagulation bekannt, so beispielsweise die bipolare Nadelelektrode, die sich unter anderem zur Myomtherapie eignet. Diese vorbekannte bipolare Elektrodenanordnung besteht aus zwei parallel angeordneten Nadelelektroden, die in das Gewebe eingestochen werden, wodurch das zwischen den Elektroden liegende Gewebe infolge des Stromflusses erhitzt und damit koaguliert wird.

Nachteilig bei den bekannten bipolaren Elektrodenanordnungen zur Tiefenkoagulation ist jedoch die relativ aufwendige Plazierung der Elektroden durch zwei Einstichstellen. Darüber hinaus läßt sich die Feldverteilung vom Anwender nur relativ ungenau vorherbestimmen, da die relative Lage der beiden Elektroden zueinander in der Regel nicht exakt vorgegeben werden kann.

Es ist aus DE 43 22 955 A1 weiterhin bekannt, zur Koagulation von Körpergewebe Laserstrahlung zu verwenden, die über einen zylindrischen Lichtwellenleiter ins Therapiegebiet übertragen werden kann, wobei der vorbekannte Lichtwellenleiter zusätzlich auch die Übertragung von Ultraschallenergie ermöglicht, so daß die beiden Therapieverfahren der ültraschall-Gewebetrennung und der Laser-Koagulation kombiniert werden können.

Aus DE 44 32 666 A1 ist weiterhin ein Wellenleiter bekannt, der neben der Übertragung von Ultraschallwellen und Laserstrahlung auch die Übertragung von Hochfrequenzenergie ermöglicht, um gleichzeitig zur Laser- und Ultraschallchirurgie auch die eingangs erwähnten Verfahren der Hochfrequenz-Chirurgie anwenden zu können. Hierzu ist der vorbekannte Wellenleiter zylindrisch ausgeführt und weist zur Übertragung der Hochfrequenzenergie zwei ebenfalls zylindrische Schichten aus elektrisch leitfähigem Material auf, die elektrisch gegeneinander isoliert sind. Der vorbekannte Wellenleiter ermöglicht somit zwar die Übertragung von Hochfrequenzenergie von einem extrakorporal angeordneten Hochfrequenzgenerator ins Therapiegebiet, gestattet jedoch keine Abgabe der Hochfrequenzenergie an das Körpergewebe.

Aus der DE 39 304 51 A1 ist eine Anordnung nach dem Oberbegriff des Anspruches 1 bekannt, bei der zwei zylindrische Elektroden beabstandet und voneinander isoliert auf einem Katheter angeordnet sind, wobei die axialen Längen der beiden Elektroden etwa.dem Katheterdurchmesser entsprechen. Dadurch lassen sich im Wesentlichen nur relativ kleine, kugelförmige Koagulationszonen im Bereich der beiden Elektroden realisieren, und da das distale Ende dieser bekannten Anordnung rund ist, ist die Einführung des Katheters in das Körpergewebe nur schwer möglich.

Aus der EP 0 530 400 A1 oder der US-A-5,007,908 ist ein Instrument für die Hochfrequenzchirurgie bekannt, bei der eine proximale Elektrode mit einem Hohlkanal versehen ist, durch den hindurch eine distale Elektrode axial ausfahrbar ist. Da die proximale Elektrode nur eine geringe axiale Länge aufweist bzw. an der Stirnfläche des proximal verlaufenden Hohlträgers ausgebildet ist, eignen sich diese bekannten Instrumente nur zur Koagulation vergleichsweise kleiner Gewebezonen.

Aus der WO 92/19167 A1 ist ein elektrochirurgisches Instrument mit einer bipolaren Elektrodenanordnung bekannt, deren beide Elektroden in geringem Abstand voneinander eine konische Spitze am distalen Ende des Katheters bilden. Da die beiden Elektroden eine gegenüber dem Katheterdurchmesser reduzierte axiale Länge besitzen, lassen sich mit dieser bekannten Anordnung nur sehr kleine kugelförmige Koagulationszonen im Bereich der Elektroden herstellen.

Der Erfindung liegt somit die Aufgabe zugrunde, eine Anordnung zur elektro-thermischen Behandlung des menschlichen oder tierischen Körpers zu schaffen, die mittels einer bipolaren Elektrodenanordnung eine interstitielle Gewebekoagulation mit vergrößenter koagulationszone ermöglicht und besonders einfach an der Behandlungsort verbracht werden kann.

Die Aufgabe wird, ausgehend von einer Anordnung gemäß dem Oberbegriff des Anspruchs 1, durch die im kennzeichnenden Teil des Anspruchs 1 angegebenen Merkmale gelöst.

Die Erfindung schließt die technische Lehre ein, zur Thermotherapie eine bipolare Elektrodenanordnung zu verwenden, bei der die beiden zylindrischen Elektroden hintereinander beabstandet auf einem langgestreckten vorne spitzen Katheter angeordnet sind, um eine gemeinsame und einfache Einführung der beiden Elektroden in den Körper durch eine einzige Einstichstelle zu ermöglichen, wobei die beiden Elektroden mit dem Katheter verbunden oder ein Bestandteil des Katheters sind.

Der Beariff Katheter ist hierbei und im folgenden allgemein zu verstehen und nicht auf die vorzugsweise verwendeten und im folgenden ausführlich beschriebenen hohlzylindrischen Anordnungen beschränkt, sondern auch mit massiven Anordnungen nahezu beliebiger Querschnitte realisierbar. Maßgebend für die erfindungsgemäße Funktion ist lediglich, daß die beiden Elektroden gemeinsam durch eine Einstichstelle in den Körper des Patienten eingeführt werden können, und wobei die axiale Länge der proximelen Elektrode um ein Mehrfaches gröber ist als die axiale Länge der distalen Elektrode. Durch den erfindungsgemäßen Katheter ist es nunmehr erstmals möglich, die Elektroden in tiefen Gewebsschichten zu plazieren und dort eine partielle, vergleichsweise großvolumige Gewebekoagulation zu erreichen.

Die Elektroden sind bei der erfindungsgemäßen Anordnung für die Zuführung der zur Gewebeerwärmung erforderlichen elektrischen Energie mit einer Stromquelle verbunden, wobei der Begriff Stromquelle nicht auf Stromquellen im engeren Sinne mit einem konstanten Strom beschränkt ist, sondern auch. die vorzugsweise verwendeten Wechselstromgeneratoren, insbesondere Hochfrecuenzgeneratoren, einschließt.

Der Abstand der beiden Elektroden ist vorgegeben, um einen einfachen Aufbau des Katheters zu erreichen und eine vorgegebene Feldverteilung sicherzustellen. Der Katheter weist hierzu ebenfalls ein langgestrecktes Trägerelement aus elektrisch isolierendem Material auf, an dessen Seite die Elektroden in axialer Richtung fixiert und zueinander beabstandet angeordnet sind. Das Trägerelement dient hierbei zum einen zur mechanischen Fixierung der Elektroden, um aufgrund des konstanten Elektrodenabstandes eine vorgegebene Feldverteilung zu erreichen. Zum anderen hat das Trägerelement die Aufgabe, die beiden Elektroden elektrisch gegeneinander zu isolieren und besteht deshalb aus elektrisch isolierendem Material. Das Trägerelement weist einen zylindrischen Querschnitt auf, wobei die beiden Elektroden bevorzugt hohlzylindrisch ausgeführt sind. Die Elektroden können hierzu beispielsweise als metallische Beschichtung auf die Oberfläche des Trägerelements aufgebracht werden oder jeweils aus einer metallischen Hülse bestehen, die auf das Trägerelement aufgeschoben wird und mit diesem eine Preßpassung bildet.

In einer anderen Ausführungsform dieser Variante wird die axiale Fixierung der Elektroden nicht durch deren Anordnung auf einem Trägerelement bewirkt, sondern durch jeweils ein Verbindungselement zwischen den Elektroden, welches die Elektroden an ihren Stirnseiten miteinander verbindet. Neben der axialen Fixierung der Elektroden hat das Verbindungselement auch die Aufgabe, die beiden Elektroden gegeneinander zu isolieren und besteht deshalb aus einem elektrisch isolierenden Material. Die Elektroden und die Verbindungsstücke sind zylindrisch und weisen denselben Querschnitt auf, so daß die Außenkontur des Katheters an den Übergangsstellen zwischen den Elektroden und den Verbindungselementen einen stetigen Verlauf ohne hervorspringende Kanten aufweist, was bei der Einführung des Katheters in den Körper des Patienten wichtig ist, um unnötige Verletzungen zu vermeiden.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: als bevorzugtes Ausführungsbeispiel der Erfindung eine Anordnung zur elektrothermischen Behandlung mit einem Katheter zur Einführung der Elektroden in den Körper und einen Handhabungsteil zur Führung des Katheters,
- Figur 2: einen Katheter mit feststehenden Elektroden zur Erreichung einer vorgegebenen Feldverteilung.

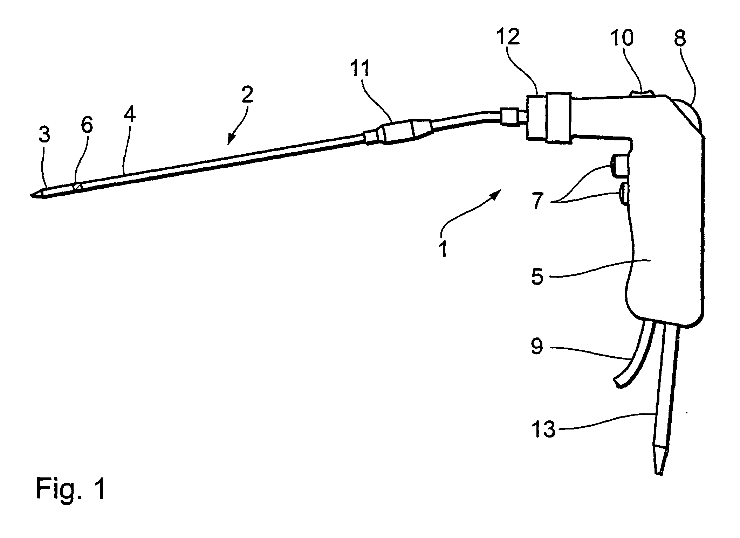

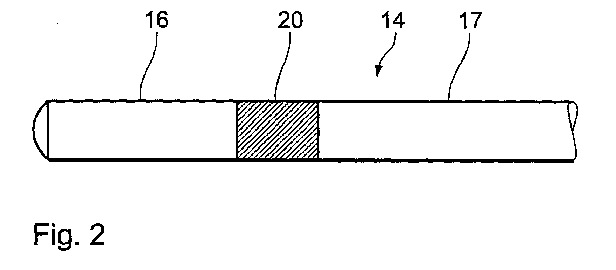

Figur 1 zeigt als bevorzugtes Ausführungsbeispiel der Erfindung eine Anordnung 1 zur elektro-thermischen Behandlung des menschlichen oder tierischen Körpers, die im wesentlichen aus einem Katheter 2 mit einer distalen Elektrode 3 und einer proximalen Elektrode 4 sowie einem Handhabungsteil 5 zur Führung des Katheters 2 besteht, wobei ein bevorzugter Katheter 2 detailliert in Figur 2 dargestellt ist.

Der Katheter 2 ermöglicht in der in Fig. 1 dargestellten Ausführungsform eine Einstellung des axialen Abstandes Zwischen den beiden Elektroden 3, 4, um die Feldverteilung im Therapiegebiet vorgeben zu können. Hierzu weist der Katheter 2 die zylindrische distale Elektrode 3 aus Edelstahl mit einem Durchmesser von 800 µm auf, die an ihrer Mantelfläche bis auf ihr distales Ende zur elektrischen Isolierung mit einer 50 µm dicken Polyimidschicht 6 versehen ist. Diese beschichtete distale Elektrode 3 liegt koaxial verschiebbar in der hohlzylindrischen proximalen Elektrode 4, die ebenfalls aus Edelstahl besteht und einen Innendurchmesser von 900 µm aufweist. Der Außendurchmesser der proximalen Elektrode 4 beträgt 1500 µm bei einer Länge von 10 cm.

Durch einen in das Handhabungsteil 5 integrierten Verschiebemechanismus läßt sich die innere Kernelektrode 3 vor dem Einstechen des Katheters 2 in das Gewebe zurückziehen, so daß die distale Elektrode 3 und die proximale Elektrode 4 zusammen einen symmetrisch angeschliffenen Einstichdorn bilden.

Die axiale Verstellbarkeit der Elektroden ist jedoch nicht Gegenstand des beanspruchten Schutzbegehrens. Die Trennung der Elektroden 3, 4 von dem Handhabungsteil 5 ermöglicht in einfacher Weise eine Sterilisierung und anschließende Wiederverwertung der Elektroden 3, 4.

Der Katheter 2 ist über ein drehbares Aufnahmelager 12 mit dem Handhabungsteil 5 verbunden und ermöglicht durch seine abgewinkelte Form ein an das Sichtfeld des Arztes angepaßtes Arbeiten, wie es zum Beispiel bei der Nasenmuschelkoagulation notwendig ist.

Weiterhin ermöglicht die dargestellte Anordnung 1 die Einleitung einer Spülflüssigkeit in das Gewebe im Therapiegebiet, um die elektrische Ankopplung zu verbessern. Hierdurch läßt sich der während der Koagulation auftretende Flüssigkeitsverlust ausgleichen, der sonst zu einer Änderung der elektrischen Impedanz des Gewebes im Therapiegebiet führt und die elektrische Ankopplung verschlechtert. Das Handhabungsteil 5 ermittelt deshalb aus der anliegenden Spannung und dem Strom über die Elektroden 3, 4 die Gewebeimpedanz und gibt eine entsprechende Menge Spülflüssigkeit an das Gewebe ab, um die Gewebeimpedanz konstant zuhalten. Die Spülflüssigkeit wird dem Handhabungsteil 5 über eine Schlauchleitung 13 von einer separaten Spülpumpe zugeführt und durch die höhle Mantelelektrode 4 ins Therapiegebiet gepumpt. Dort tritt die Spülflüssigkeit dann durch einen Spalt zwischen der distalen Elektrode 3 und der proximalen Elektrode 4 in das Gewebe aus.

Die Figur 2 zeigt jeweils einen Katheter 14. mit einer proximalen Elektrode 17 und einer distalen Elektrode 16, wobei der Abstand zwischen den beiden Elektroden 16, 17 konstant ist, um eine vorgegebene Feldverteilung zu erreichen und einen einfachen Aufbau des Katheters 14, zu ermöglichen. Die beiden Elektroden 16, 17 sind hierbei zylindrisch ausgeführt und werden an ihren Stirnflächen durch ein ebenfalls zylindrisches Verbindungsstück 20 des Katheters 14 aus elektrisch isolierendem Material mechanisch mit einander verbunden, wobei das Verbindungsstück 20 wie auch die proximale Elektrode 17 zur Aufnahme der Elektrodenzuleftung einen axial verlaufenden Hohlkanal aufweist. Bei dem in Figure 2a dargestellten Katheter 14 sind die Außenquerschnitte der beiden Elektroden 16, 17 und der Querschnitt des Verbindungsstück 20 gleich, so daß die Außenkontur des Katheters 14 auch an den Übergangsstellen zwischen den Elektroden 16, 17 und dem Verbindungsstück 20 glatt ist, wodurch das Einführen des Katheters 14 in den Körper des Patienten erleichtert wird.

## Patentansprüche

1. Anordnung zur elektro-thermischen Behandlung des menschlichen oder tierischen Körpers, insbesondere zur Gewebekoagulation oder Elektrotomie,
mit zwei zylindrischen Elektroden (16, 17) zur Einführung in den zu behandelnden Körper, wobei die beiden Elektroden (16, 17) zur Erzeugung eines das Körpergewebe im Behandlungsgebiet erwärmenden elektrischen oder elektromagnetischen Feldes elektrisch gegeneinander isoliert und zueinander beabstandet angeordnet sowie über jeweils eine Zuleitung mit einer extrakorporal angeordneten Stromquelle verbunden sind,
wobei zur gemeinsamen Einführung der beiden zylindrischen Elektroden (16, 17) in den Körper ein langgestreckter zylindrischer Katheter (14) vorgesehen ist,
wobei die proximale Elektrode (17) in axialer Richtung des Katheters (14) gegen die distale Elektrode (16) versetzt angeordnet ist,
wobei die beiden Elektroden (16, 17) mit dem Katheter (14) verbunden oder ein Bestandteil des Katheters sind und einen konstanten axialen Abstand voneinander aufweisen,
und wobei der Aussenquerschnitt des Katheters (14) längs der beiden Elektroden (16, 17) gleich dem Aussenquerschnitt zwischen den beiden Elektroden (16, 17) ist,
und wobei die axiale Länge der proximalen Elektrode (17) um ein Mehrfaches größer ist als die axiale Länge der distalen Elektrode (16),
**dadurch gekennzeichnet**,
dass der Katheter (14) zur Erleichterung der Einführung in den Körper eine sich zu seinem distalen Ende hin verjüngende Spitze aufweist,
und das die axiale Länge der Elektroden (16, 17) um ein Mehrfaches größer ist als der axiale Abstand zwischen den Elektroden (16, 17).

2. Anordnung nach Anspruch 1, **dadurch gekennzeichnet, dass** der Katheter (14) zur mechanischen Verbindung der beiden Elektroden (16, 17) miteinander ein Verbindungsstück (20) aus elektrisch isolierendem Material aufweist, das zwischen den beiden Elektroden (16, 17) angeordnet ist und diese an ihren Stirnseiten miteinander verbindet.

3. Anordnung nach einem der vorhergehenden Ansprüche, **dadurch gekennzeichnet, dass** die proximale Elektrode (17) und die distale Elektrode (16) zur Zuführung einer Spülflüssigkeit ins Therapiegebiet und/oder zur Plazierung eines Temperatursensors oder eines Lichtwellenleiters einen entlang ihrer Längsachse verlaufenden Hohlkanal aufweist.

4. Anordnung nach Anspruch 3, **dadurch gekennzeichnet, dass** zur Abgabe der Spülflüssigkeit im Therapiegebiet in der Wandung der proximalen Elektrode (17) und/oder der distalen Elektrode mindestens ein Durchbruch und/oder zwischen der proximalen Elektrode (17) und der distalen Elektrode (16) mindestens am distalen Ende der proximalen Elektrode (17) ein Spalt vorgesehen ist.

## Claims

1. An arrangement for electrothermal treatment of the human or animal body, in particular for tissue coagulation or electrotomy, having two cylindrical electrodes (16,17) for insertion into the body to be treated, where the two electrodes (16,17) are electrically insulated from one another and are arranged spaced from one another to produce an electric or electromagnetic field heating the body tissue in the treatment area, and are each connected by a feed line with a current source arranged outside the body,
and where an elongate cylindrical catheter (14) is provided for the joint insertion of the two cylindrical electrodes (16,17) into the body,
where the proximal electrode (17) is arranged staggered from distal electrode (16) in the axial direction of catheter (14),
where the two electrodes (16, 17) are connected to catheter (14) or a component thereof and have a constant axial distance from one another,
and whereby the outer cross-section of catheter (14) along the two electrodes (16,17) is identical to the outer cross-section between the two electrodes (16,17),
and where the axial length of proximal electrode (17) is several times larger than the axial length of distal electrode (16), **characterised in that**
catheter (14) has a point tapering towards its distal end for ease of insertion into the body,
and that the axial length of electrodes (16,17) is several times larger than the axial distance between electrodes (16, 17).

2. An arrangement according to claim 1, **characterised in that** catheter (14) has a connecting part (20) made from electrically insulating material, for mechanically connecting the two electrodes (16,17) with one another, which is arranged between the two electrodes (16,17) and connects them with one another at their end surfaces.

3. An arrangement according to any of the above claims, **characterised in that** proximal electrode (17) and distal electrode (16) have a hollow channel extending along their longitudinal axis for supplying a flushing fluid into the therapy area and/or for placing a temperature sensor or a light waveguide into same.

4. An arrangement according to claim 3, **characterised in that** there is at least a break through in the wall of proximal electrode (17) and/or distal electrode, and/or between proximal electrode (17) and distal electrode (16) there is at least a gap for the release of flushing fluid into the therapy area.

## Revendications

1. Système pour le traitement électrothermique du corps humain ou animal, en particulier pour la coagulation des tissus, ou pour l'électrotomie,
comprenant deux électrodes cylindriques (16, 17) pour l'introduction dans le corps à traiter, lesdites deux électrodes (16, 17) étant isolées électriquement l'une par rapport à l'autre et agencées à distance l'une de l'autre pour la production d'un champ électrique ou électromagnétique qui réchauffe les tissus corporels dans la zone de traitement, et lesdites électrodes étant reliées l'une à l'autre par une ligne d'amenée respective à une source électrique agencée à l'extérieur du corps,
dans lequel un cathéter cylindrique allongé (14) est prévu pour l'introduction conjointe des deux électrodes cylindriques (16, 17),
les électrodes proximales (17) étant agencées de manière décalée par rapport à l'électrode distale (16) en direction axiale du cathéter (14),
les deux électrodes (16, 17) étant reliées au cathéter (14) ou constituent un composant du cathéter et présentent une distance axiale constante l'une de l'autre,
la section extérieure du cathéter (14) le long des deux électrodes (16, 17) est égale à la section extérieure entre les deux électrodes (16, 17),
et la longueur axiale de l'électrode proximale (17) est supérieure d'un multiple à la longueur axiale de l'électrode distale (16),
**caractérisé en ce que**
le cathéter (14) comporte une pointe qui va en se rétrécissant en direction de son extrémité distale pour faciliter l'introduction dans le corps, et
la longueur axiale des électrodes (16, 17) est supérieure d'un multiple à la distance axiale entre les électrodes (16, 17).

2. Système selon la revendication 1, **caractérisé en ce que** le cathéter (14) comprend, pour la liaison mécanique des deux électrodes (16, 17) l'une à l'autre, une pièce de liaison (20) en matériau électriquement isolant, agencé entre les deux électrodes (16, 17) et reliant celles-ci l'une à l'autre au niveau de leurs faces frontales.

3. Système selon l'une des revendications précédentes, **caractérisé en ce que** l'électrode proximale (17) et l'électrode distale (16) comprennent, pour l'admission d'un liquide de rinçage dans la zone de thérapie et/ou pour la mise en place d'un capteur de température ou d'un guide d'ondes, un canal creux qui s'étend le long de leur axe longitudinal.

4. Système selon la revendication 3, **caractérisé en ce que** pour la sortie du liquide de rinçage dans la zone de thérapie, il est prévu au moins une traversée dans la paroi de l'électrode proximale (17) et/ou de l'électrode distale, et/ou une fente au moins à l'extrémité distale de l'électrode proximale (17) entre l'électrode proximale (17) et l'électrode distale (16).
